# EUROPÄISCHE PATENTSCHRIFT

(11) **EP 0 780 667 B1**
(45) Veröffentlichungstag und Bekanntmachung des Hinweises auf die Patenterteilung: **24.10.2001**
(21) Anmeldenummer: 96120134.0
(22) Anmeldetag: 14.12.1996
(51) Int. Cl.: G01G 17/04, A61B 5/20

(54) **Urinmessgerät**
Device for measuring urine
Appareil de mesure d'urine

(30) Priorität: 23.12.1995 DE 19548678
(43) Veröffentlichungstag der Anmeldung: 25.06.1997
(73) Patentinhaber: Sarstedt AG & Co., 51588 Nümbrecht (DE)
(72) Erfinder: Köttig, Thomas, 69151 Neckargemünd (DE)
(74) Vertreter: Pollmeier, Felix, Dipl.-Ing.

(56) Entgegenhaltungen:
- GB-A- 1 533 204
- GB-A- 2 207 902
- US-A- 4 699 155
- US-A- 4 712 567

## Beschreibung

Die Erfindung betrifft ein Urinmeßgerät, das sich z.B. am Krankenbett eines Patienten befestigen läßt, zum Überwachen und Erfassen des Urinflusses eines katheterisierten Patienten, insbesondere zum Ermitteln des Urin-Volumens, umfassend ein Urinsammelbehältnis, an das ein Katheterschlauch und ein weichelastischer Abflußschlauch angeschlossen sind.

Ein Gerät dieser Art ist durch die DE 43 38 687 C1 bekanntgeworden. Es wird vor allem auf Intensivstationen in Krankenhäusern eingesetzt, wo es notwendig ist, den Flüssigkeitshaushalt eines Patienten zu bilanzieren. Zu diesem Zweck wird die dem Patienten beispielsweise mittels Infusionen zugeführte Flüssigkeitsmenge erfaßt und mit den Flüsigkeitsmengen verglichen die der Patient auf natürlichem ege oder über künstliche Kreisläufe abgibt. Die hier interessierende, wichtigste Körperflüssigkeit ist der Harn bzw. Urin, der auf Intensivstationen üblicherweise aus der Blase des Patienten abgeführt wird, indem ein Katheterschlauch in die Blase des Patienten geschoben wird. An den Katheter schließt sich ein Abflußschlauch an, durch den der Urin in ein unterhalb des Patienten am Bett befestigtes Sammelgefäß, in der Regel ein Urinbeutel, abläuft. Damit sich eine retrograde Verkeimung der Schlauchleitung und damit eine Infektion des Patienten verhindern lassen, ist vorzugsweise innerhalb der Schlauchleitung zumeist eine Tropfkammer angeordnet, durch die der Verkeimungsweg unterbrochen wird. Zu den wichtigsten aussagekräftigen Meßwerten, an denen ein diagnostizierender Arzt im Zusammenhang mit Harn interessiert ist, gehört das Gesamtvolumen des innerhalb eines bestimmten Zeitraumes abgelassenen Harnes, wie beispielsweise der sogenannte 24-Stunden-Harn, der durchschnittliche Volumenfluß an Harn innerhalb eines bestimmten Zeitintervalls, die spezifische Dichte des Harns sowie eine optische Kontrolle der Farbe des Urins.

Bei der Urinmessung muß verhindert werden, daß äußere Einflüsse die Ermittlung des Volumens beeinträchtigen. Der Katheterschlauch wird daher in der Regel nicht direkt - was gleichwohl möglich wäre - mit dem Urinsammelbehältnis verbunden, sondern über die zwischengeschaltete Tropfkammer, die zudem der Zugentlastung dient. Damit auf den Abflußschlauch wirkende Kräfte, beispielsweise aufgrund einer Bewegung des Patienten, nicht zu einer das Meßergebnis verfälschenden Krafteinleitung in den Abflußschlauch führen, ist der Abflußschlauch bei dem bekannten Urinmeßgerät so lang, daß eine im wesentlichen kraftfreie Schlauchschlaufe entsteht. Das setzt jedoch eine sorgfältige Auswahl des Materials für den Abflußschlauch voraus, das nämlich auf keinen Fall zu steif sein darf. Wenn das Material aber zu weich ist, der Abflußschlauch z.B. aus Latex besteht, hat sich gezeigt, daß ein solcher Schlauch durch äußere Einflüsse, wie ein unabsichtlicher Berührungskontakt des Meßgerätes durch das Pflegepersonal, zum Abknicken neigt und zusammenklappt. Eine Urinableitung bzw. ein Urinfluß von der Tropfkammer zu dem Sammelbehältnis findet dann nicht mehr statt. Es entsteht vielmehr ein Rückstau, der das Rückschlagventil in der Tropfkammer schließt. Nachströmender Urin kann folglich nicht mehr aus der Tropfkammer fließen und füllt zuerst die Tropfkammer und anschließend den Katheterschlauch.

Um meßwertverfälschende Einflüsse bei der Ermittlung des Urin-Gesamtvolumens zu vermeiden, ist es aus der DE 35 44 031 A1 bekannt, eine Meßkammeranordnung und eine Entkopplungsvorrichtung als Verbindung zwischen dem distalen Ende eines in den Körper eines Patienten eingesetzten Katheters und dem Urinsammelbehälter vorzusehen. Der Katheter ist mit einem Katheteranschlußteil an ein Schlauchanschlußteil eines elastischen Abflußschlauches aus Kunststoff angeschlossen. Die Entkopplungsvorrichtung besitzt eine elastische Manschette, durch die eine Übertragung von auf den Abflußschlauch einwirkenden Druck- und Stoßkräften auf den Sammelbehälter verhindert werden. Hierzu ist es erforderlich, die Entkopplungsvorrichtung in spezieller Weise in Eingriff mit Befestigungsarmen zu bringen, die das Urinsammelbehältnis (Auffangbeutel) schwebend halten. Eine solche Entkopplungsvorrichtung erfordert somit einen entsprechend hohen Aufwand.

GB 1 533 204 A und GB 2 207 902 A zeigen Anordnungen, die das Knicken von Abflußschläuchen bei Urinmeßgeräten verhindern.

Der Erfindung liegt die Aufgabe zugrunde, ein gattungsgemäßes Urinmeßgerät einfacher und dennoch betriebssicherer zu gestalten.

Diese Aufgabe wird erfindungsgemäß durch ein koaxial in dem Abflußschlauch langgestreckt angeordnetes, flexibles, eine flüssigkeitsumströmbare, unebene Außenkontur aufweisendes Schlauch-Innenelement gelöst. Es wird hierbei von der Überlegung ausgegangen, daß eine wie vorbeschrieben aufwendige Entkopplungsvorrichtung entfallen und stattdessen mit einem mit Sicherheit keine meßwertverfälschenden Kräfte übertragenden Abflußschlauch aus weichem Material gearbeitet werden kann, wenn trotz des Einsatzes eines weichelastischen Schlauches ein unbeschadet von äußeren Einflüssen stetiger Urin-Abfluß gewährleistet werden kann. Diese Voraussetzung wird durch das erfindungsgemäß in dem aus einem weichen Material bestehenden Abflußschlauch koaxial angeordnete, eine unebene Außenkontur aufweisende Schlauch-Innenelement geschaffen, das nämlich bei einer aufgrund von äußeren Einwirkungen entstehenden Knickstelle des Abflußschlauches eine Unterbrechung des Urinflusses verhindert. Die Formgebung mit einer unebenen bzw. unregelmäßigen Außenkontur des Schlauch-Innenelementes schließt aus, daß der elastische, weiche Abflußschlauch, sollte es zu einer Knickstelle kommen, das Schlauch-Innenelement auf dem gesamten Umfang berührt; die Innenwandung des Verbindungsschlauches liegt dem Schlauch-Innenelement vielmehr lediglich partiell an, ist folglich stets umströmbar und hält damit den Abfluß des nachfließenden Urins aufrecht.

Das Schlauch-Innenelement, bei dem es sich beispielsweise um eine Perlenkette, einen länglichen Streifen aus weichem Kunststoff oder Gummi mit in regelmäßigen oder unregelmäßigen Abständen vorgesehenen Einschnürungen oder einen Gegenstand mit einer schrauben- oder gewindeförmigen Außenkontur handeln kann, läßt sich von einer solchen Länge vorsehen, daß es bis zu dem Urinsammelbehältnis reicht. Durch das die Bewegungen des weichelastischen Abflußschlauches mitmachende Schlauch-Innenelement wird weiterhin eine dauerhafte Verklebung der Knickstelle vermieden, die ansonsten durch im Urin vorhandene Bestandteile begünstigt wird. Es ist auf jeden Fall stets ein solch großer Querschnitt des Abflußschlauches frei, daß der Urin mit Sicherheit abfließen kann und ein nachteiliger Rückstau ausgeschlossen wird.

Weitere Einzelheiten und Vorteile der Erfindung ergeben sich aus dem Patentbegehren und der nachfolgenden Beschreibung, in der ein in den Zeichnungen dargestelltes Ausführungsbeispiel des Gegenstandes der Erfindung im Zusammenhang mit einem eine Tropfkammer aufweisenden Urinmeßgerät näher erläutert ist. Es zeigen:
- Fig. 1: im Teilschnitt eine Gesamtansicht eines Urinmeßgerätes; und
- Fig. 2: als Einzelheit einen die Tropfkammer des Urinmeßgerätes mit einem Urinbeutel verbindenden, koaxial ein Schlauch-Innenelement aufnehmenden Abflußschlauch.

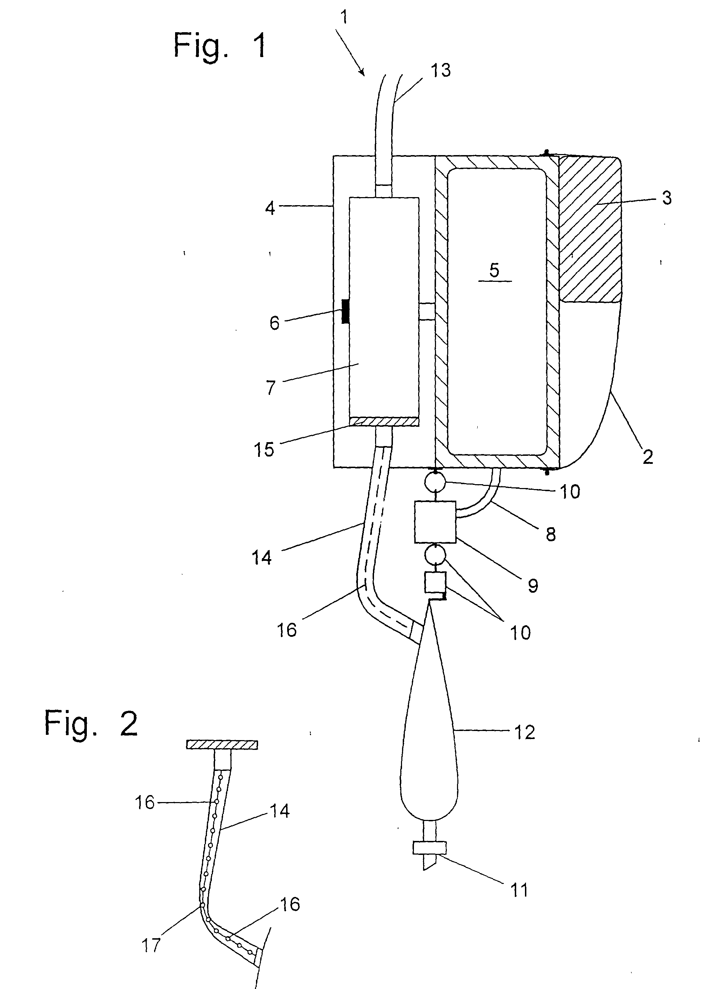

Das in Fig. 1 gezeigte Urinmeßgerät 1 ist hier mittels mindestens einem Halteband 2 an einem Bettenholm 3 eines Krankenbettes befestigt. Das Gerätegehäuse 4 besitzt einen Raum 5 für einen Rechner mit Anzeige-Einrichtung und nimmt weiterhin eine eingesetzte und mittels einer Klemmeinheit 6 gesicherte Tropfkammer 7 auf. Von dem Raum 5 der Rechner- und Anzeigeeinrichtung führt ein Meßkabel 8 zu einer Meßeinheit 9, an die über eine Aufhängung 10 ein an seinem Auslaßende mit einem Ablaßhahn 11 versehener Urinbeutel 12 angehängt ist. In die Tropfkammer 7 mündet von oben ein Katheterschlauch 13 ein, und das untere Ende der Tropfkammer 7 ist über einen aus einem weichelastischen Material bestehenden Abflußschlauch 14 mit dem Urinbeutel 12 verbunden. Eine bodenseitig in der Tropfkammer 7 angeordnete Rückflußsperre 15 verhindert, daß Urin aus dem Urinbeutel 12 und/oder dem Abflußschlauch 14 zurücktrömt.

Wie sich näher aus Fig. 2 entnehmen läßt, ist in dem Abflußschlauch 14 zwischen der Tropfkammer 7 und dem Urinbeutel 12 ein langgestrecktes, flexibles, im Ausführungsbeispiel als Perlenkette ausgebildetes Schlauch-Innenelement 16 angeordnet, das vom unteren Ende der Tropfkammer 7 bzw. von unterhalb der Rücklaufsperre 15 bis zu dem Einlaß bzw. in die Nähe des Einlassers des Urinbeutels 12 reicht oder gar in diesem eintaucht. Das Schlauch-Innenelement 16 ist ebenso biegsam und weichelastisch wie der ebenfalls kraftübertragungsfrei angeordnete weiche Abflußschlauch 14. Sofern die im wesentlichen kraftfreie Schlauchschlaufe 'des Abflußschlauches 14 aufgrund unbeabsichtigter äußerer Einflüsse, z.B. hervorgerufen durch Bewegungen des Patienten in seinem Krankenbett oder Berührungen des Urinmeßgerätes 1 durch das Pflegepersonal, abknicken und eine Knickstelle 17 bilden sollte, wie in Fig. 2 gezeigt, so verhindert das Schlauch-Innenelement 16, daß sich die einander gegenüberliegenden Innenwandungen des Abflußschlauches 14 berühren, was bei bekannten Meßeinrichtungen zu einer dauerhaften Verklebung der Knickstelle des Abflußschlauches 14 führt; die Schlauchinnenwandung legt sich vielmehr lediglich partiell an die unregelmäßige bzw. unebene Außenkontur des Schlauch-Innenelementes 16 an. Es ist damit sichergestellt, daß der Urin aus der Tropfkammer 7 auch weiter in den Urinbeutel 12 abfließt, denn das flüssigkeitsumströmbar, z.B. mit einer unebenen Außenkontur oder eckig, ausgebildete Schlauch-Innenelelement 16 hält den Durchströmungsquerschnitt des Abflußschlauches 14 in einer ausreichenden Größe stets offen und kann von dem Urin umströmt werden, der keinen Rückstau in der Tropfkammer 7 und dem zum Patienten führenden Katheterschlauch 13 bewirken kann.

## Patentansprüche

1. Urinmeßgerät, das sich z.B. am Krankenbett eines Patienten befestigen läßt, zum Überwachen und Erfassen des Urinflusses eines katheterisierten Patienten, insbesondere zum Ermitteln des Urin-Volumens, umfassend ein Urinsammelbehältnis, an das ein Katheterschlauch und ein weichelastischer Abflußschlauch angeschlossen sind,
**gekennzeichnet durch**
ein koaxial in dem Abflußschlauch (14) langgestreckt angeordnetes, flexibles, eine flüssigkeitsumströmbare, unebene Außenkontur aufweisendes Schlauch-Innenelement (16).

## Claims

1. Urine measuring instrument, which can be mounted at, for example, the sickbed of a patient, for monitoring and detecting the urine flow of a catheterised patient, especially for ascertaining the volume of urine, comprising a urine collecting container with which a catheter hose and a soft-elastic outflow hose are connected, **characterised by** a flexible hose inner element (16) which is arranged extended in length coaxially in the outflow hose (14), can be flowed around by liquid and has an uneven outer contour.

## Revendications

1. Appareil de mesure d'urine, qui peut par exemple être fixé au lit d'un malade, pour la surveillance et l'enregistrement du flux urinaire d'un patient portant un cathéter, en particulier pour la détermination du volume d'urine, comprenant un récipient de collecte d'urine, auquel sont raccordés un flexible de cathéter et un flexible d'évacuation souple et élastique, **caractérisé par** un élément interne de flexible (16) coaxial, disposé longitudinalement dans le flexible d'évacuation (14), flexible, présentant un contour externe rugueux, pouvant être entouré de liquide.
